# EUROPEAN PATENT APPLICATION

(11) **EP 3 742 489 A1**
(43) Date of publication of application: **25.11.2020**
(21) Application number: 18899823.1
(22) Date of filing: 17.10.2018
(51) Int. Cl.: H01L 27/146, A61B 5/02, A61B 5/026, A61B 5/1171, A61B 5/1172, G06T 1/00, H04N 5/369

(54) **LIVING BODY INFORMATION ACQUISITION DEVICE, LIVING BODY INFORMATION ACQUISITION METHOD AND WEARABLE DEVICE**

(30) Priority: 15.01.2018 JP 2018004015
(71) Applicant: Sony Corporation, 108-0075 Tokyo (JP)
(72) Inventor: KOBAYASHI, Naoko, Tokyo 108-0075 (JP); SAWANO, Ryosuke, Tokyo 108-0075 (JP); SATO, Yusuke, Tokyo 108-0075 (JP)
(74) Representative: MFG Patentanwälte Meyer-Wildhagen Meggle-Freund Gerhard PartG mbB
(86) International application number: PCT/JP2018/038583
(87) International publication number: WO 2019/138633

(57) **Abstract**

[Problem] To enable holding down the occurrence of stray light to a greater extent even in the case of attempting further downsizing and thinning of the device.

[Solution] A biological information obtaining device according to the application concerned includes an image sensor in which biological information, which represents image information obtained as a result of emitting the light of a predetermined wavelength with respect to one part of a living body, is imaged; a lens array that is positioned in between the image sensor and one part of the living body, that has a plurality of single lenses arranged in an array-like manner, and that performs imaging of the biological information in the image sensor; and light shields that are positioned in between the image sensor and the lens array, and that form light guiding paths meant for imaging of the biological information, which is transmitted through the single lenses, in the image sensor. The light guiding paths are formed to have unequal opening diameters along the surface normal direction of the image sensor.

## Description

### Field

The application concerned is related to a biological information obtaining device, a biological information obtaining method, and a wearable device.

### Background

In recent years, in order to guarantee the information security of mobile information devices, the biological authentication technology that makes use of biological information, such as fingerprint pattern images and vein pattern images, is becoming increasingly significant. At the same, there is advancement in the technological development of mobile information devices, and measures are being taken to make the mobile information devices thinner. Accordingly, also regarding biological information obtaining devices that obtain biological information mentioned above, there is a demand for achieving downsizing and thinning.

In that regard, in Patent Literature 1 mentioned below, a technology has been proposed in which thinning of an imaging device is attempted by installing a light focusing/shielding element, which includes two lens arrays and a single aperture array, at a stage prior to a light detecting element.

### Citation List

### Patent Literature

Patent Literature 1: Japanese Patent Application Laid-open No. 2011-203792

### Summary

### Technical Problem

In the case of attempting further thinning of a biological imaging device; the light guiding path, which guides the light constituting biological information such as fingerprint pattern images or vein pattern images to an imaging device, becomes shorter in length by necessity. When the light guiding path becomes shorter in length, it becomes more likely to have the occurrence of stray light. At the same time, when a biological imaging device is further downsized, there is no alternative but to shorten the lens pitch of the lens array as used in Patent Literature 1 mentioned above, and it becomes more likely to have the occurrence of stray light due to the shortening of the lens pitch. In this way, in the case of attempting further downsizing and thinning of a biological imaging device, it becomes more likely to have the occurrence of stray light due to the abovementioned two factors.

In view of the issues mentioned above, in the application concerned, a biological information obtaining device, a biological information obtaining method, and a wearable device are proposed that enable holding down the occurrence of stray light to a greater extent even in the case of attempting further downsizing and thinning of the device.

### Solution to Problem

According to the present disclosure, a biological information obtaining device is provided that includes: an image sensor in which biological information, which represents image information obtained as a result of emitting light of predetermined wavelength with respect to one part of a living body, is imaged; a lens array that is positioned in between the image sensor and the one part of the living body, has a plurality of single lenses arranged in an array-like manner, and performs imaging of the biological information in the image sensor; and a light shield that is positioned in between the image sensor and the lens array, and forms a light guiding path meant for imaging of the biological information, which is transmitted through each of the single lenses, in the image sensor, wherein the light guiding path is formed to have unequal opening diameter along surface normal direction of the image sensor.

Moreover, according to the present disclosure, a biological information obtaining method is provided that includes performing imaging of biological information, which represents image information obtained as a result of emitting light of predetermined wavelength with respect to one part of a living body, using a lens array, which has a plurality of single lenses arranged in an array-like manner, in an image sensor via a light guiding path formed by a light shield, wherein the light guiding path is formed to have unequal opening diameter along surface normal direction of the image sensor.

Moreover, according to the present disclosure, a wearable device is provided that includes: a device main body that is wearable by user; and a biological information obtaining device that is installed in the device main body, wherein the biological information obtaining device includes an image sensor in which biological information, which represents image information obtained as a result of emitting light of predetermined wavelength with respect to one part of a living body, is imaged, a lens array that is positioned in between the image sensor and the one part of the living body, has a plurality of single lenses arranged in an array-like manner, and performs imaging of the biological information in the image sensor, and a light shield that is positioned in between the image sensor and the lens array, and forms a light guiding path meant for imaging of the biological information, which is transmitted through each of the single lenses, in the image sensor, and the light guiding path is formed to have unequal opening diameter along surface normal direction of the image sensor.

According to the application concerned, the biological information passes through the lens array and via the light guiding paths, and is imaged in the image sensor. At that time, since the light guiding paths are formed to have unequal opening diameters, the occurrence of stray light is held down.

### Advantageous Effects of Invention

As described above, according to the application concerned, the occurrence of stray light can be held down to a greater extent even in the case of attempting further downsizing and thinning of devices.

The abovementioned effect is not necessarily limited in scope and, in place of or in addition to the abovementioned effect, any other effect indicated in the present written description or any other effect that may occur from the present written description can also be achieved.

### Brief Description of Drawings

FIG. 1A is a cross-sectional view that schematically illustrates an example of the cross-sectional structure of a biological information obtaining device according to an embodiment of the application concerned.
FIG. 1B is a cross-sectional view that schematically illustrates another example of the cross-sectional structure of the biological information obtaining device according to the embodiment.
FIG. 2 is an explanatory diagram for explaining about the biological information obtaining device according to the embodiment.
FIG. 3 is an enlarged cross-sectional view that schematically illustrates a portion of the cross-sectional surface of the biological information obtaining device according to the embodiment.
FIG. 4 is an explanatory diagram for explaining about the overlapping of images due to a lens array.
FIG. 5 is an explanatory diagram for explaining about light shields included in the biological information obtaining device according to the embodiment.
FIG. 6 is an explanatory diagram for explaining about the light shields included in the biological information obtaining device according to the embodiment.
FIG. 7 is an explanatory diagram for explaining about the light shields included in the biological information obtaining device according to the embodiment.
FIG. 8 is an explanatory diagram for explaining about the light shields included in the biological information obtaining device according to the embodiment.
FIG. 9 is an enlarged cross-sectional view that schematically illustrates a portion of the cross-sectional surface of the biological information obtaining device according to the present embodiment.
FIG. 10A is an explanatory diagram for explaining about the biological information obtaining device according to the embodiment.
FIG. 10B is an explanatory diagram for explaining about the biological information obtaining device according to the embodiment.

### Description of Embodiments

A preferred embodiment of the application concerned is described below in detail with reference to the accompanying drawings. In the present written description and the drawings, the constituent elements having practically identical functional configuration are referred to by the same reference numerals, and the explanation is not given repeatedly.

The explanation is given in the following sequence.
1. Embodiment
   1.1. Regarding structure of biological information obtaining device
   1.2. Regarding method for manufacturing biological information obtaining device
   1.3. Regarding biological information obtaining method
   1.4. Implementation examples of biological information obtaining device

### (Embodiment)

### <Regarding structure of biological information obtaining device>

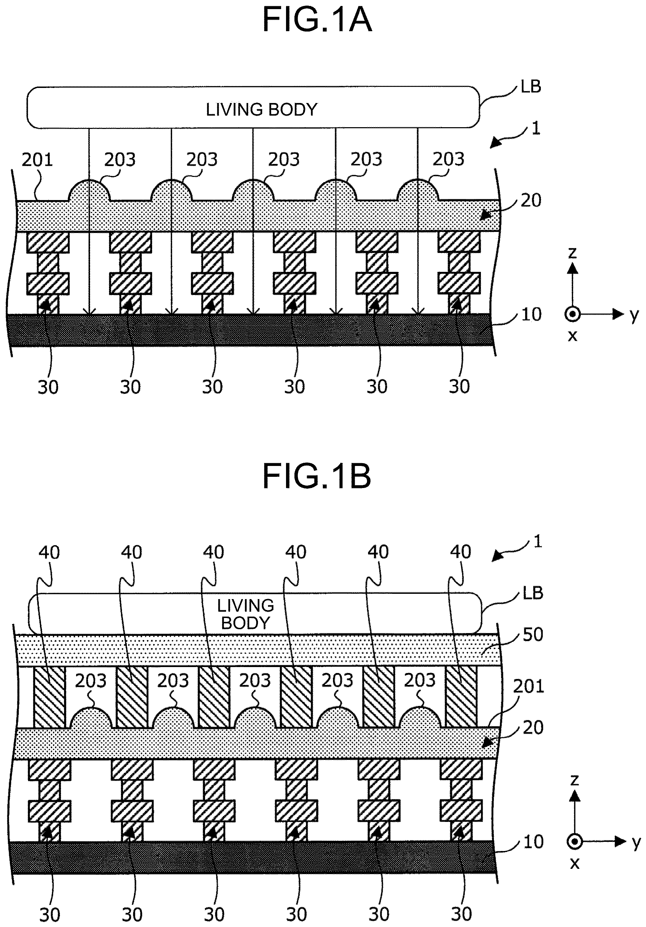

Firstly, explained below in detail with reference to FIGS. 1A to 9 is a structure of a biological information obtaining device according to the embodiment of the application concerned.

The biological information obtaining device according to the present embodiment is a device for obtaining biological information that represents image information obtained as a result of irradiating one part of a living body with the light having a predetermined wavelength. Although there is no particular restriction on the biological information, examples thereof include image information related to fingerprints (for example, fingerprint pattern images indicating the distribution of the fingerprints); image information related to perspiration (for example, images indicating the presence or absence of perspiration); image information related to blood flow; image information related to pulse wave or pulsation; and image information related to blood vessels (for example, vein pattern images indicating the distribution of veins).

The biological information obtaining device according to the present embodiment is not only capable of obtaining one type of such variety of biological information, but is also capable of simultaneously obtaining a plurality of types of biological information. The type of biological information to be obtained can be arbitrarily selected by appropriately adjusting the wavelength of the light to be emitted onto one part of the living body or adjusting the type of the part of the living body from which the image information is to be obtained. As a result, different types of biological information can be obtained in combination, such as obtaining the biological information related to fingerprints in combination with the biological information related to perspiration. Moreover, when the biological information obtained by the biological information obtaining device is to be used in various known biological authentication technologies, the authentication can be performed by combining different types of biological information, so that the authentication accuracy can be further enhanced.

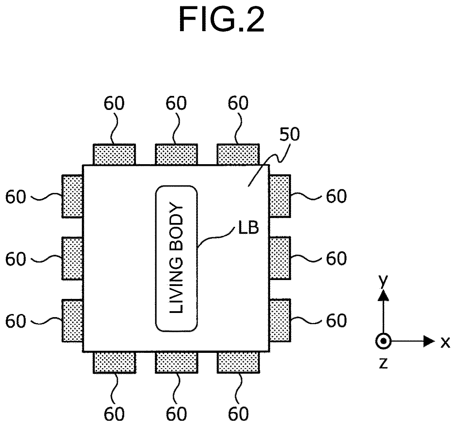

FIG. 1A is a cross-sectional view that schematically illustrates an example of the cross-sectional structure of the biological information obtaining device according to the present embodiment that is capable of obtaining biological information as explained above. FIG. 1B is a cross-sectional view that schematically illustrates another example of the cross-sectional structure of the biological information obtaining device according to the present embodiment. FIG. 2 is an explanatory diagram for explaining about the biological information obtaining device according to the present embodiment.

As schematically illustrated in FIG. 1A, a biological information obtaining device 1 according to the present embodiment includes an image sensor 10 in which biological information representing image information obtained from a living body LB is imaged; a lens array 20 that is positioned in between the image sensor 10 and one part of the living body LB; and light shields 30 that are positioned in between the image sensor 10 and the lens array 20 and that form the light guiding paths for the purpose of imaging the biological information in the image sensor 10.

The lens array 20 is configured by arranging a plurality of single lenses 203 in an array-like manner on a predetermined base member 201; and performs imaging of the biological information, which is transmitted through the single lenses 203, in the pixels provided in the image sensor 10. As schematically illustrated in FIG. 1A, the space between each pair of neighboring light shields 30 functions as a light guiding path. In the present embodiment, as schematically illustrated in FIG. 1A, the light guiding paths formed by the light shields 30 are formed in such a way that their opening diameters (widths in the y-axis direction of the light guiding paths with reference to FIG. 1A) are not equal along the surface normal direction of the image sensor 10 (along the z-axis direction in FIG. 1A).

Meanwhile, in addition to the configuration illustrated in FIG. 1A, for example, as illustrated in FIG. 1B, the biological information obtaining device 1 according to the present embodiment desirably includes the following: second-type light shields 40 that form second-type light guiding paths in between the lens array 20 and one part of the living body LB for the purpose of guiding, in a sequential manner, the light representing the biological information from the side of the lens array 20 toward the single lenses 203; and a cover glass 50 having one part of the living body LB placed on its outer surface.

Moreover, for example, as illustrated in FIG. 2, the biological information obtaining device 1 according to the present embodiment desirably includes light source units 60 that are arranged around the cover glass 50 and that irradiate one part of the living body LB with the light which has a predetermined wavelength and which is used in obtaining the biological information.

Given below is the detailed explanation of each constituent element of the biological information obtaining device 1 according to the present embodiment.

### [Regarding image sensor 10]

On that surface of the image sensor 10 which is facing the lens array 20, a plurality of pixels of the image sensor 10 is arranged; and the concerned surface functions as the sensor surface of the image sensor 10. The biological information obtained from the living body LB is imaged as a result of the imaging performed on the sensor surface of the image sensor 10, and substantive image data related to the biological information is generated.

As long as the image sensor 10 is an imaging device configured with pixels having sensitivity with respect to the wavelength of the light constituting the biological information, it is possible to use any known type of imaging device. Examples of such an imaging device include a CCD (Charge Coupled Devices) sensor and a CMOS (Complementary Metal Oxide Semiconductor) sensor. Meanwhile, the image sensor 10 according to the present embodiment can either be a color sensor or be a monochromatic sensor.

The pixels constituting the image sensor 10 desirably have the pixel size that enables taking images of objects having the line width in the range of a few µm to a few hundred µm. If p[µm] represents the size of the photographic subject (for example, one part of a living body, such as fingerprints or veins) and if M represents the magnification of the single lenses 203 included in the lens array 20, then p×M[µm] becomes the size of the photographic subject on the sensor surface. The pixels constituting the image sensor 10 desirably have such a pixel size which enables expressing the size of the photographic subject on the sensor surface with two or more pixels. Examples of such a pixel size include the pixel size in the range of 1 µm² to 4 µm². However, the pixel size in the image sensor 10 according to the present embodiment is not limited to that example.

Since the pixels constituting the image sensor 10 have the pixel size that enables taking images of objects having the line width in the range of a few µm to a few hundred µm; it becomes possible to generate biological information having the resolution of 1000 PPI (Pixel Per Inch) or more, for example. Meanwhile, regarding the pixels constituting the image sensor 10, smaller the pixel size, the higher is the possibility of generating high-definition biological information. Hence, it is desirable to have a small pixel size as much as possible. Also regarding the resolution of the generated biological information, since it is desirable to have high-definition resolution as much as possible, the upper limit value of the resolution is not particularly specified.

### [Regarding lens array 20]

As explained earlier, the lens array 20 has a plurality of single lenses 203 arranged in an array-like manner on the predetermined base member 201. The lens array 20 is also called a microlens array.

Regarding the material used for the base member 201 and the single lenses 203, as long as it is possible to transmit the light constituting the biological information, there is no particular restriction on the material and any material usable as the material for optical devices can be used. Examples of such material include various types of resin such as thermoplastic resin or thermosetting resin typified by acrylic resin, polycarbonate resin, or polyolefin resin; and various types of optical glass. Such material can be subjected to molding, pattern embossing, or lithography according to a known method; and the desired shape of the lens array can be achieved.

There is no particular restriction on the lens shape of the single lenses 203, and it is possible to have spherical single lenses 203 or aspherical single lenses 203.

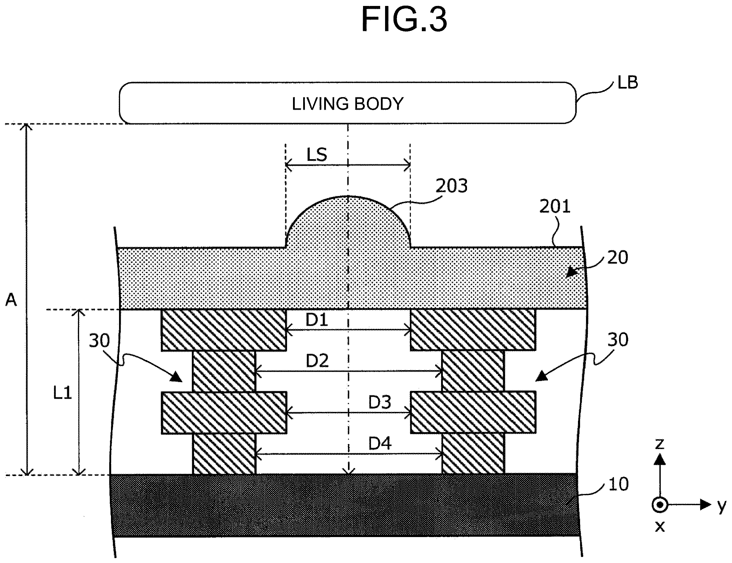

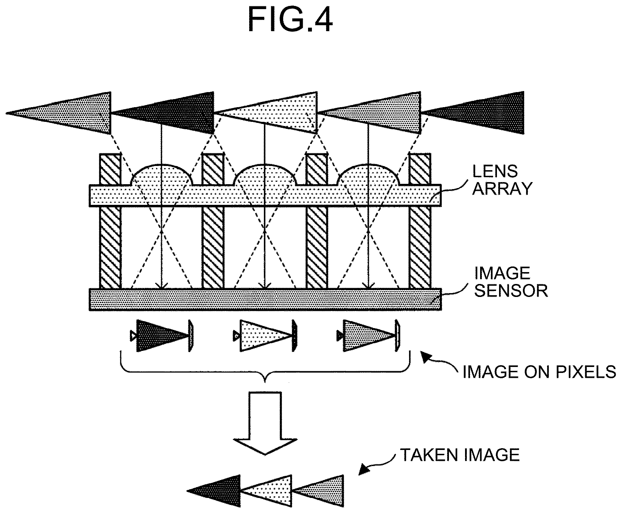

FIG. 3 is an enlarged cross-sectional view that schematically illustrates a portion of the cross-sectional surface of the biological information obtaining device 1 according to the present embodiment. FIG. 4 is an explanatory diagram for explaining about the overlapping of images due to the lens array.

In the lens array 20 according to the present embodiment, regarding the lens diameter of the single lenses 203 (a length LS with reference to FIG. 3), it is preferable to have the lens diameter to be as large as possible with respect to the pixel size of the image sensor 10. That is, having a large number of pixels in each single lens 203 implies having a smaller total number of the single lenses 203 in the lens array 20. Hence, not only it becomes possible to reduce the number of operations accompanying image synthesis explained below with reference to FIG. 4, but it also becomes possible to ensure that there is overlapping. At that same time, in order to achieve downsizing of the device, if it is attempted to shorten the distance between the sensor surface of the image sensor 10 and one part of the living body LB (i.e., if it is attempted to shorten the focal length of the single lenses 203), it becomes important to make the lens diameter LS of the single lenses 203 smaller. In such a situation too, it is desirable that the lens diameter LS of the single lenses 203 is equal to or greater than 20 times the pixel size of the image sensor 10.

As illustrated in FIG. 4, generally, in a lens array, the light constituting images is guided to the sensor surface of the image sensor in such a way that not only the object positioned directly above each single lens is included but some part of the neighboring objects is also included. With reference to FIG. 4, it is not only the triangular object positioned directly above each single lens that is imaged in the image sensor, but some part of the right-side end of the triangular object positioned directly above the left neighboring single lens as well as some part of the left-side end of the triangular object positioned directly above the right neighboring single lens is also imaged in the image sensor. In this way, as a result of performing imaging while overlapping some part of the images that should be formed in the neighboring pixels, it becomes possible to take images having good contrast.

If the lens diameter LS of the single lenses 203 illustrated in FIG. 3 is smaller than 20 times the pixel size of the image sensor 10, then there is insufficient amount of overlapping (insufficient number of pixels contributing to overlapping) as explained with reference to FIG. 4, and it is likely that high-definition images (i.e., biological information of a higher degree of accuracy) cannot be obtained. By setting the lens diameter LS of the single lenses 203 to be equal to or greater than 20 times the pixel size of the image sensor 10, it becomes possible to obtain biological information of a higher degree of accuracy. Meanwhile, there is no particular restriction on the upper limit value of the lens diameter LS with respect to the pixel size of the image sensor 10, and the lens diameter LS can be appropriately decided by taking into account the amount of overlapping.

In order to ensure that overlapping occurs as explained with reference to FIG. 4, it is important that the lens magnification of the single lenses 203 is smaller than one times. On the other hand, when the lens magnification of the single lenses 203 becomes equal to or greater than one times, overlapping cannot be ensured and it becomes highly unlikely to be able to obtain high-definition images. Meanwhile, although the amount of overlapping increases in inverse proportion to the lens magnification of the single lenses 203; if the Nyquist limit is taken into account, it becomes important to decide on the lens magnification M in such a way that the relationship s×M>2×N is established. In that relational expression, s represents the minimum value of the photographic subject size to be resolved; M represents the lens magnification; and N represents the pixel size of the image sensor.

The distance from the sensor surface of the image sensor 10 to one part of the living body LB (i.e., a distance A with reference to FIG. 3), which gets set because of the lens array 20 including the single lenses 203, is desirably equal to or greater than 0.5 mm and smaller than 30 mm. If the distance A is smaller than 0.5 mm, then the manufacturing difficulty is more likely to increase. On the other hand, if the distance A is equal to or greater than 30 mm, then it is likely that downsizing of the biological information obtaining device 1 is insufficient. Thus, as a result of keeping the distance A equal to or greater than 0.5 mm and smaller than 30 mm, it becomes possible to further downsize the device while holding down on the increase in the manufacturing difficulty.

Meanwhile, each single lens 203 that constitutes the lens array 20 as explained above can be a lens group configured by combining a plurality of lenses. Moreover, it is also possible to install an objective lens in between the lens array 20 and the living body LB representing the photographic subject.

### [Regarding light shields 30]

As explained earlier, the light shields 30 are positioned in between the image sensor 10 and the lens array 20, and form light guiding paths meant for forming images of the biological information in the image sensor 10. Regarding the material of the light shields 30, there is no particular restriction, and at least either various metals such as stainless steel, or various resin composition materials, or various dielectric substances including silicon can be used.

In the biological information obtaining device 1 according to the present embodiment, as schematically illustrated in FIGS. 1A, 1B, and 3, the light guiding paths representing the spaces formed by the pairs of neighboring light shields 30 have unequal opening diameters D along the surface normal direction of the image sensor 10 (i.e., along the z-axis direction).

That is, as illustrated in FIG. 3, the opening diameters of the light guiding paths do not have the equal value among them, and there is a portion having an opening diameter D1, a portion having an opening diameter D2, a portion having an opening diameter D3, and a portion having an opening diameter D4. When the opening diameters of the light guiding paths are equal, the light incident from the portion other than the single lenses 203, which are positioned directly above the light guiding paths, (i.e., the stray light) becomes more likely to be subjected to regular-reflection from the wall surface of the light guiding paths and to reach the image sensor 10. However, for example, as illustrated in FIG. 3, if the opening diameters of the light guiding paths have unequal values thereby making the wall surfaces of the light guiding paths have an uneven shape, even if the incident stray light is reflected from the wall surface of the light guiding paths, the stray light intensity goes on attenuating while getting repeatedly reflected from the wall surface of the light guiding paths, and thus the possibility of the post-reflection stray light reaching the image sensor 10 can be reduced. As a result, in the biological information obtaining device 1 according to the present embodiment, even in the case of attempting further downsizing and thinning of the device, it becomes possible to further hold down the occurrence of stray light.

More specifically, the opening diameter D is desirably equal to or greater than twice the wavelength of the light constituting the biological information, and is desirably equal to or smaller than four times the lens diameter LS of the single lenses 203. If the opening diameter D is smaller than twice the wavelength of the light, then it becomes more likely to have a diffraction phenomenon of the light in the light guiding paths, and noise is likely to get superimposed on the biological information converted into images in the image sensor 10. On the other hand, if the opening diameter is greater than four times the lens diameter LS of the single lenses 203, it is likely to have an increase in the stray light falling in the light guiding paths. Thus, within the range in which the abovementioned conditions are satisfied, the opening diameter D of the light guiding path can be varied in various ways along the surface normal direction of the image sensor 10 (i.e., along the z-axis direction).

However, if the opening diameter D of the light guiding paths is reduced to be smaller than the lens diameter LS of the single lenses 203, it not only becomes possible to exclude the effect of the end portions of such single lenses 203 which are likely to have declined in lens characteristics, but it also becomes easier to hold down the stray light coming from the neighboring light guiding paths. That enables achieving improvement in the obtained image quality. On the other hand, if the opening diameter D of the light guiding path becomes small, although it results in the narrowing of the angle of view, it becomes important to shorten the center-to-center distance (what is called the lens pitch) of the neighboring single lenses 203 for the purpose of overlapping images in the neighboring single lenses 203. Hence, at the time of manufacturing the lens array 20 having a narrow lens pitch, a higher degree of accuracy is sometimes demanded. In that context, the opening diameters D of the light guiding paths are desirably set to be equal to or greater than 0.5 times the lens diameter LS of the single lenses 203. When the opening diameters D of the light guiding paths are greater than the lens diameter LS of the single lenses 203, the amount of overlapping also increases. At the same time, when the opening diameters D of the light guiding paths are greater than the lens diameter LS of the single lenses 203, the effect of the end portions of such single lenses 203 which are likely to have declined in lens characteristics becomes conspicuous, and there is an increase in the possibility that stray light falls from the neighboring light guiding paths. That leads to a demand for making the light shields 30 thinner, and a higher degree of accuracy is sometimes demanded. In that context, the opening diameters D of the light guiding paths are more desirably set to be equal to or greater than 0.5 times the lens diameter LS of the single lenses 203 and equal to or smaller than four times the lens diameter LS of the single lenses 203.

In the biological information obtaining device 1 according to the present embodiment, the light path length of the light guiding paths formed by the pair of neighboring light shields 30 (i.e., a length L1 with reference to FIG. 3) is desirably shorter than the length of the back focal distance of the lens array 20. If A [mm] represents the distance from the sensor surface of the image sensor 10 to the living body LB and if M represents the lens magnification of the single lenses 203, the length of the back focal distance of the lens array 20 is expressed as (A×M)/(1×M) [mm]. Thus, it is desirable that the light path length L1 of the light guiding paths is shorter than (A×M)/(1×M) [mm]. When the light path length L1 of the light guiding paths satisfies the abovementioned conditions, it becomes possible to obtain higher-definition biological information.

As illustrated in FIGS. 1A, 1B, and 3; each light shield 30 can be a laminated body in which two or more light shielding members having a predetermined shape are laminated along the surface normal direction of the image sensor 10 (i.e., along the z-axis direction). Alternatively, each light shield 30 can be formed using a single light shielding member.

When the light shields 30 are cut parallel to the surface normal direction of the image sensor 10 (i.e., parallel to the z-axis direction), the outside shape of the light shields 30 facing the light guiding paths can be formed either by straight lines or by curved lines as illustrated in FIGS. 1A, 1B, and 3. Alternatively, when the light shields 30 are cut parallel to the surface normal direction of the image sensor 10 (i.e., parallel to the z-axis direction), the outside shape of the light shields 30 facing the light guiding paths can be formed by straight lines and curved lines.

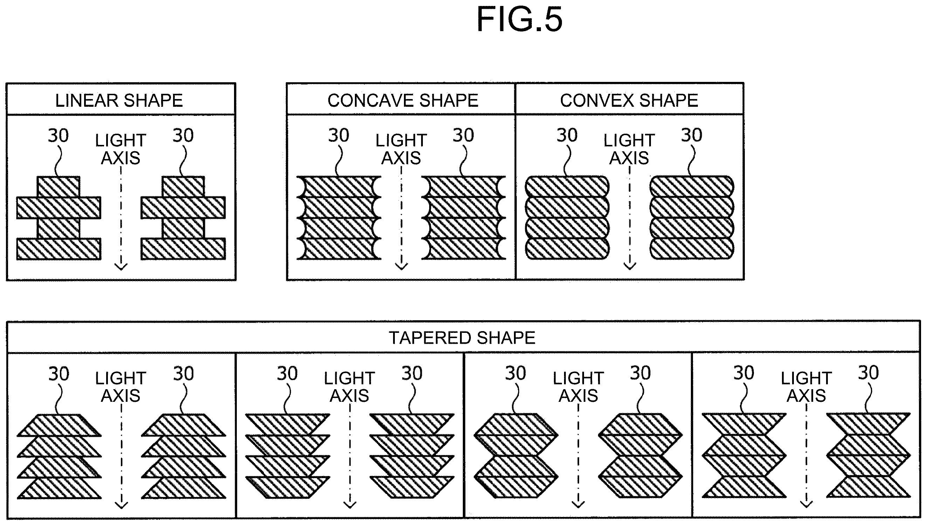

Explained below in brief with reference to specific examples illustrated in FIGS. 5 to 8 are examples of the outside shape of the light shields 30. FIGS. 5 to 8 are explanatory diagrams for explaining about the light shields included in the biological information obtaining device according to the present embodiment.

According to the present embodiment, as illustrated in the top left portion of FIG. 5, the light shields 30 facing the light guiding paths can have the outside shape as a linear shape made by straight lines that are substantially parallel to the surface normal direction of the image sensor 10 (i.e., parallel to the z-axis direction). Alternatively, as illustrated in the lower portion of FIG. 5, the light shields 30 can have the outside shape as a tapered shape made by straight lines that are not parallel to the surface normal direction of the image sensor 10 (i.e., not parallel to the z-axis direction). When the light shields 30 have the outside shape as a linear shape as illustrated in FIG. 5, in the light guiding paths formed by the pairs of neighboring light shields 3, the opening diameter changes in a discontinuous manner along the surface normal direction of the image sensor 10 (i.e., along the z-axis direction). On the other hand, when the light shields 30 have the outside shape as a tapered shape as illustrated in FIG. 5, in the light guiding paths formed by the pairs of neighboring light shields 3, the opening diameter changes in a continuous manner along the surface normal direction of the image sensor 10 (i.e., along the z-axis direction).

Alternatively, the light shields 30 facing the light guiding paths can have the outside shape as a concave shape or a convex shape as illustrated in the top right portion of FIG. 5. Regarding the light shields 30 having such a concave shape or a convex shape, the outside shape facing the light guiding paths is made of curved lines as illustrated in the top right portion of FIG. 5. In the case of the concave shape or the convex shape as illustrated in FIG. 5, in the light guiding paths formed by the pairs of neighboring light shields 3, the opening diameter changes in a continuous manner along the surface normal direction of the image sensor 10 (i.e., along the z-axis direction).

Meanwhile, the outside shape of the light shields 30 is not limited to the examples illustrated in FIG. 5, and it is possible to arbitrarily combine light shielding members having a rectangular shape, light shielding members having a tapered shape, and light shielding members having a convex shape or a concave shape.

In FIG. 5 is illustrated the case in which four light shielding members having a predetermined shape are laminated. However, the lamination can be of two or three layers, or can be of five or more layers.

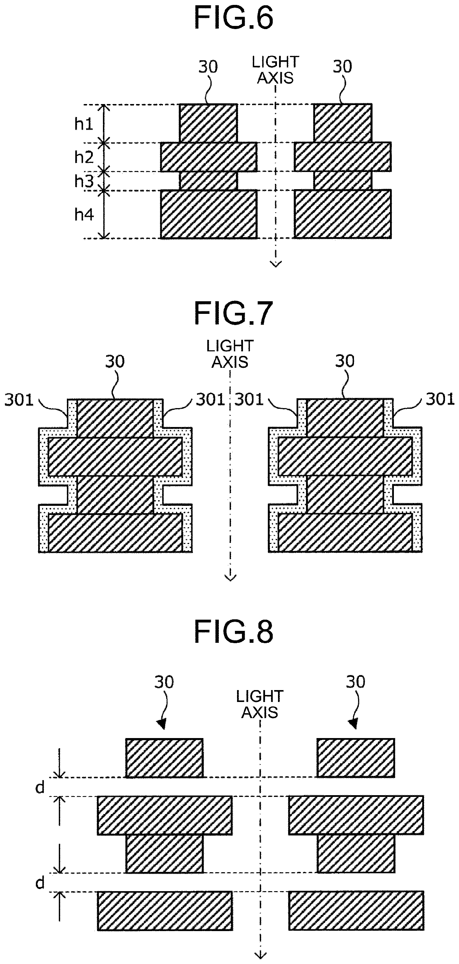

As illustrated in FIG. 5, the height (the length in the z-axis direction) of each light shielding member either can be identical as illustrated in FIG. 5 or can be different as illustrated in FIG. 6. In FIG. 6 is illustrated the case in which, in the light shields 30 made of four laminated layers, all light shielding members have different heights h1 to h4. If it is possible to ensure that the opening diameters of the light guiding paths are not equal along the z-axis direction, there is no particular restriction on the combination of the heights h of the light shielding members.

As illustrated in FIG. 7, in the present embodiment, on the outer surface of the light shields 30 facing the light guiding paths, it is desirable to dispose an absorber 301 that has the light absorptivity of 90% or higher at a predetermined wavelength (for example, the light absorptivity of 90% or higher at the wavelength of the light constituting the biological information or the light absorptivity of 90% or higher at the wavelength of the light emitted for obtaining the biological information). As a result of disposing the absorber 301, every time the light that falls in the light guiding paths as stray light reaches the wall surfaces of the light shields 30, it gets absorbed by the absorber 301. As a result, it becomes possible to reliably reduce the stray light reaching the image sensor 10, and to obtain higher-definition biological information. Regarding the material of the absorber 301, there is no particular restriction, and any known material can be used by taking into account the wavelength of the light to be absorbed.

Moreover, in the present embodiment, on the outer surfaces of the light shields 30 facing the light guiding paths, for example, it is possible to perform a variety of regular reflection prevention processing such as forming minute uneven shapes, or performing plating with a metal having a predetermined surface roughness. As a result of performing such regular reflection prevention processing, it becomes possible to reliably reduce the stray light reaching the image sensor 10, and to obtain higher-definition biological information. Meanwhile, if the absorber 301 is disposed as well as regular reflection prevention processing is performed; it becomes possible to further reliably reduce the stray light reaching the image sensor 10, and to obtain still higher-definition biological information.

In the explanation given above, for example, as illustrated in FIG. 3, the example is given in which light shielding members having a predetermined shape are laminated without any gaps in between. Alternatively, for example, as illustrated in FIG. 8, in the case of forming each light shield 30 as a laminated body of light shielding members having a predetermined shape, it is possible to keep a gap d of a predetermined thickness in between the neighboring light shielding members.

### [Regarding second-type light shields 40]

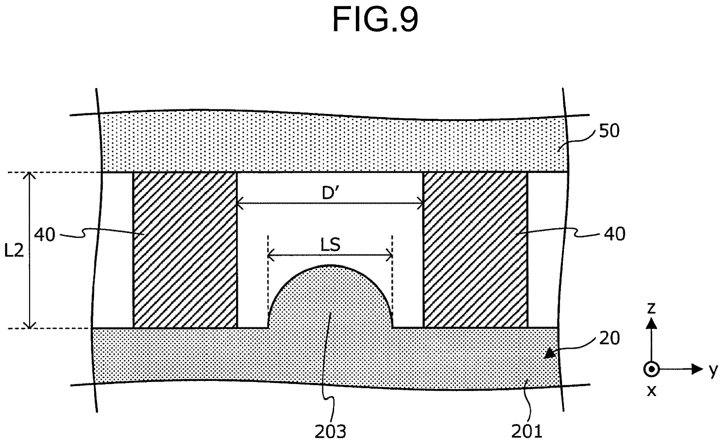

Explained below in detail with reference to FIGS. 1B and 9 are the second-type light shields 40. FIG. 9 is an enlarged cross-sectional view that schematically illustrates a portion of the cross-sectional surface of the biological information obtaining device according to the present embodiment.

In the biological information obtaining device 1 according to the present embodiment, as illustrated in FIG. 1B, it is desirable to further dispose the second-type light shields 40 above the lens array 20 on the side of the single lenses 203. Thus, each pair of neighboring second-type light shields 40 constitutes a second-type light guiding path meant for guiding the light constituting biological information from one part of the living body LB to the corresponding single lens 203. As a result of disposing the second-type light shields 40 as illustrated in FIG. 1B, the light constituting the biological information can be more reliably guided to the single lenses 203, as well as incidence of stray light onto the single lenses 203 is prevented.

In an identical manner to the light shields 30, the second-type light shields 40 too can be formed using at least either various metals such as stainless steel, or various resin composition materials, or various dielectric substances including silicon.

Although there is no particular restriction on the height of the second-type light shields 40 (i.e., a length L2 in the z-axis direction with reference to FIG. 9), since the second-type light shields 40 function as diaphragms, it is desirable to have a low height as much as possible. Thus, the height can be appropriately decided by taking into account the ease of manufacturing the second-type light shields 40 and the quality of the biological information to be obtained.

Moreover, it is desirable that the second-type light guiding paths, which are formed due to the pairs of neighboring second-type light shields 40, have an opening diameter D' set to be greater than but close to the lens diameter LS of the single lenses 203 (i.e., D'>LS holding true). If the opening diameter D' of the second-type light guiding paths is equal to the lens diameter LS of the single lenses 203, it sometimes becomes difficult to achieve overlapping that was explained earlier. At the same time, closer the opening diameter D' of the second-type light guiding paths to the lens diameter LS of the single lenses 203, the more it becomes possible to further reliably prevent incidence of stray light onto the single lenses 203.

### [Regarding cover glass 50]

As illustrated in FIGS. 1B and 9, it is desirable to have the cover glass 50 disposed in between the second-type light shields 40 and one part of the living body LB for the purpose of protecting the constituent elements of the biological information obtaining device 1. Herein, one part of the living body LB gets placed on the cover glass 50.

Regarding the material used for the cover glass 50, as long as it is possible to transmit the light constituting the biological information, there is no particular restriction on the material, and any material usable as the material for optical devices can be used. Examples of such material include various types of resin such as thermoplastic resin or thermosetting resin typified by acrylic resin, polycarbonate resin, or polyolefin resin; and various types of optical glass. However, from the perspective of strength and impact resistance, it is desirable to form the cover glass 50 using various types of resin.

Regarding the thickness of the cover glass 50, there is no particular restriction and the thickness can be appropriately set.

In FIGS. 1B and 9 is illustrated the case in which the cover glass 50 is disposed to make contact with the second-type light shields 40. Alternatively, it is also possible to have some airspace (i.e., a gap) between the second-type light shields 40 and the cover glass 50). Moreover, in FIGS. 1B and 9 is illustrated the case in which the biological information obtaining device 1 according to the present embodiment further includes the second-type light shields 40 as well as the cover glass 50. Alternatively, the biological information obtaining device 1 according to the present embodiment can be configured to not include the second-type light shields 40 but to include the cover glass 50.

### [Regarding light source units 60]

As schematically illustrated in FIG. 2, it is desirable to have the light source units 60 disposed around the cover glass 50 for the purpose of emitting, onto one part of the living body LB, the light having a predetermined wavelength to be used in obtaining biological information. There is no particular restriction on the light source units 60 and, as long as the light having the required wavelength for obtaining biological information can be emitted, it is possible to use various types of known light sources such as various diodes or semiconductor lasers.

The count and the arrangement of the light source units 60 is not limited to the example illustrated in FIG. 6, and can be appropriately set according to the size of the part to be measured in the living body LB and the size of the biological information obtaining device 1. Moreover, the arrangement positions of the light source units 60 are not limited to the positions illustrated in FIG. 2, and it is possible arrange them at arbitrary positions.

Till now, detailed explanation was given with reference to FIGS. 1A to 9 about the structure of the biological information obtaining device 1 according to the present embodiment.

### <Regarding method for manufacturing biological information obtaining device 1>

Regarding the method for manufacturing the biological information obtaining device 1 according to the present embodiment, there is no particular restriction. Thus, the biological information obtaining device 1 according to the present embodiment can be manufactured according to any appropriate method including a known method used in semiconductor manufacturing processes.

For example, the image sensor satisfying the conditions explained earlier is prepared, and the light shields 30 according to the present embodiment are formed on the image sensor 10 according to a known method used in semiconductor manufacturing processes. Moreover, the lens array 20 satisfying the conditions explained earlier can be separately manufactured according to a known method, and can be placed on top of the light shields 30.

Then, as may be necessary, the second-type light shields 40 and the cover glass 50 are placed above the lens array 20. In this way, the biological information obtaining device 1 according to the present embodiment can be manufactured.

### <Regarding biological information obtaining method>

As explained above, using the biological information obtaining device 1 in which the light guiding paths are formed to have unequal opening diameters along the surface normal direction of the image sensor; biological information, which represents image information obtained as a result of emitting the light of a predetermined wavelength onto one part of the living body LB, is imaged in the image sensor 10 using the lens array 20, which has a plurality of single lenses 203 arranged in an array-like manner, via the light guiding paths formed due to the light shields 30. As a result, for example, it becomes possible to accurately obtain a variety of biological information such as image information related to fingerprints (for example, fingerprint pattern images indicating the distribution of the fingerprints); image information related to perspiration (for example, images indicating the presence or absence of perspiration); image information related to blood flow; image information related to pulse wave or pulsation; and image information related to blood vessels (for example, vein pattern images indicating the distribution of veins).

### <Implementation examples of biological information obtaining device 1>

The biological information obtaining device 1 according to the present embodiment can be implemented in any known type of device that performs biological authentication using biological information and provides various services according to the authentication result. In the biological information obtaining device 1 according to the present embodiment, since the occurrence of stray light is held down to a greater extent, it becomes possible to obtain biological information with a higher degree of accuracy. As a result of using the obtained biological information, authentication of individual users can be performed with a higher degree of accuracy.

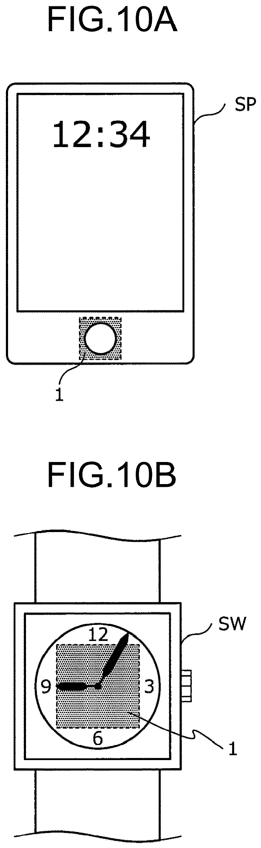

For example, the biological information obtaining device 1 according to the present embodiment can be installed in a mobile device such as a smartphone SP illustrated in FIG. 10A, a tablet terminal, a portable music player, or a portable gaming console; or can be installed in a wearable device such as a smartwatch SW illustrated in FIG. 10B; or can be installed in various types of stationary devices. Moreover, the biological information obtaining device 1 according to the present embodiment can be installed in an external device that is attachable to various stationary devices.

In the case of installing the biological information obtaining device 1 according to the present embodiment in a wearable device, it is installed inside the device main body configured to be wearable by the user (for example, inside the main body of the smartwatch SW illustrated in FIG. 10B). Herein, there is no particular restriction on the installation position of the biological information obtaining device 1, and it can be installed at an arbitrary position.

Although the application concerned is described above in detail in the form of a preferred embodiment with reference to the accompanying drawings; the technical scope of the application concerned is not limited to the embodiment described above. That is, the application concerned is to be construed as embodying all modifications such as other embodiments, additions, alternative constructions, and deletions that may occur to one skilled in the art that fairly fall within the basic teaching herein set forth.

Moreover, the effects described in the present written description are only explanatory and exemplary, and are not limited in scope. That is, in addition to or in place of the effects described above, the technology disclosed in the application concerned enables achieving other effects that may occur to one skilled in the art.

Meanwhile, a configuration as explained below also falls within the technical scope of the application concerned.
(1) A biological information obtaining device comprising:
   an image sensor in which biological information, which represents image information obtained as a result of emitting light of predetermined wavelength with respect to one part of a living body, is imaged;
   a lens array that
      is positioned in between the image sensor and the one part of the living body,
      has a plurality of single lenses arranged in an array-like manner, and
      performs imaging of the biological information in the image sensor; and
   a light shield that
      is positioned in between the image sensor and the lens array, and
      forms a light guiding path meant for imaging of the biological information, which is transmitted through each of the single lenses, in the image sensor, wherein
   the light guiding path is formed to have unequal opening diameter along surface normal direction of the image sensor.
(2) The biological information obtaining device according to (1), wherein opening diameter of the light guiding path changes in a continuous manner along the surface normal direction.
(3) The biological information obtaining device according to (1), wherein opening diameter of the light guiding path changes in a discontinuous manner along the surface normal direction.
(4) The biological information obtaining device according to any one of (1) to (3), wherein, when the light shield is cut parallel to the surface normal direction, outside shape of the light shield facing the light guiding path is formed by at least either straight lines or curved lines.
(5) The biological information obtaining device according to any one of (1) to (4), wherein the light shield is a laminated body in which two or more light shielding members having predetermined shape are laminated along the surface normal direction.
(6) The biological information obtaining device according to any one of (1) to (5), wherein opening diameter of the light guiding path is equal to or greater than twice wavelength of light constituting the biological information.
(7) The biological information obtaining device according to any one of (1) to (6), wherein opening diameter of the light guiding path is equal to or greater than 0.5 times and equal to or smaller than four times lens diameter of the single lenses.
(8) The biological information obtaining device according to any one of (1) to (7), wherein, when A [mm] represents distance from sensor surface of the image sensor to the one part of the living body and when M represents lens magnification of the single lenses, length of the light guiding path is smaller than (A×M)/(1×M) [mm].
(9) The biological information obtaining device according to any one of (1) to (8), wherein distance from sensor surface of the image sensor to the one part of the living body is equal to or greater than 0.5 mm and smaller than 30 mm.
(10) The biological information obtaining device according to any one of (1) to (10), wherein
   lens diameter of the single lenses is equal to or greater than 20 times pixel size of the image sensor,
   lens magnification of the single lenses is smaller than one times, and
   when s represents minimum value of photographic subject size to be resolved, when M represents lens magnification, and when N represents pixel size of the image sensor, relationship s×M>2×N is established.
(11) The biological information obtaining device according to any one of (1) to (10), wherein
   in between the lens array and the one part of the living body,
   a second-type light shield is disposed that forms a second-type light guiding path meant for guiding, in a sequential manner, light constituting the biological information from side of the lens array toward the single lenses, and
   a cover glass is disposed, the one part of the living body being placed on surface of the cover glass, and
   light source unit for emitting light of the predetermined wavelength onto the one part of the living body is disposed around the cover glass.
(12) The biological information obtaining device according to any one of (1) to (11), wherein an absorber having light absorptivity of 90% or higher at a predetermined wavelength is disposed on surface of the light shield facing the light guiding path.
(13) The biological information obtaining device according to any one of (1) to (12), wherein regular reflection prevention processing is performed on surface of the light shield facing the light guiding path.
(14) The biological information obtaining device according to any one of (1) to (13), wherein material of the light shield is made of at least either metal, or resin compound, or dielectric substance.
(15) The biological information obtaining device according to any one of (1) to (14), wherein the biological information indicates at least either image information related to fingerprints, or image information related to perspiration, or image information related to blood flow, or image information related to pulse wave or pulsation, or image information related to blood vessels.
(16) The biological information obtaining device according to any one of (1) to (15), wherein the biological information indicates at least two types of image information selected from among image information related to fingerprints, image information related to perspiration, image information related to blood flow, image information related to pulse wave or pulsation, and image information related to blood vessels.
(17) The biological information obtaining device according to any one of (1) to (16), wherein the biological information obtaining device is installed in a mobile device, a wearable device, or a stationary device, or is installed in an external device attachable to a stationary device.
(18) A biological information obtaining method comprising performing imaging of biological information, which represents image information obtained as a result of emitting light of predetermined wavelength with respect to one part of a living body, using a lens array, which has a plurality of single lenses arranged in an array-like manner, in an image sensor via a light guiding path formed by a light shield, wherein
   the light guiding path is formed to have unequal opening diameter along surface normal direction of the image sensor.
(19) A wearable device comprising:
   a device main body that is wearable by user; and
   a biological information obtaining device that is installed in the device main body, wherein
   the biological information obtaining device includes
      an image sensor in which biological information, which represents image information obtained as a result of emitting light of predetermined wavelength with respect to one part of a living body, is imaged,
      a lens array that
         is positioned in between the image sensor and the one part of the living body,
         has a plurality of single lenses arranged in an array-like manner, and
         performs imaging of the biological information in the image sensor, and
      a light shield that
         is positioned in between the image sensor and the lens array, and
         forms a light guiding path meant for imaging of the biological information, which is transmitted through each of the single lenses, in the image sensor, and
   the light guiding path is formed to have unequal opening diameter along surface normal direction of the image sensor.

### Reference Signs List

- 1: biological information obtaining device
- 10: image sensor
- 20: lens array
- 30: light shield
- 40: second-type light shield
- 50: cover glass
- 60: light source unit
- 201: base material
- 203: single lens
- 301: absorber

## Claims

1. A biological information obtaining device comprising:
an image sensor in which biological information, which represents image information obtained as a result of emitting light of predetermined wavelength with respect to one part of a living body, is imaged;
a lens array that
is positioned in between the image sensor and the one part of the living body,
has a plurality of single lenses arranged in an array-like manner, and
performs imaging of the biological information in the image sensor; and
a light shield that
is positioned in between the image sensor and the lens array, and
forms a light guiding path meant for imaging of the biological information, which is transmitted through each of the single lenses, in the image sensor, wherein
the light guiding path is formed to have unequal opening diameter along surface normal direction of the image sensor.

2. The biological information obtaining device according to claim 1, wherein opening diameter of the light guiding path changes in a continuous manner along the surface normal direction.

3. The biological information obtaining device according to claim 1, wherein opening diameter of the light guiding path changes in a discontinuous manner along the surface normal direction.

4. The biological information obtaining device according to claim 1, wherein, when the light shield is cut parallel to the surface normal direction, outside shape of the light shield facing the light guiding path is formed by at least either straight lines or curved lines.

5. The biological information obtaining device according to claim 1, wherein the light shield is a laminated body in which two or more light shielding members having predetermined shape are laminated along the surface normal direction.

6. The biological information obtaining device according to claim 1, wherein opening diameter of the light guiding path is equal to or greater than twice wavelength of light constituting the biological information.

7. The biological information obtaining device according to claim 1, wherein opening diameter of the light guiding path is equal to or greater than 0.5 times and equal to or smaller than four times lens diameter of the single lenses.

8. The biological information obtaining device according to claim 1, wherein, when A [mm] represents distance from sensor surface of the image sensor to the one part of the living body and when M represents lens magnification of the single lenses, length of the light guiding path is smaller than (A×M)/(1×M) [mm].

9. The biological information obtaining device according to claim 1, wherein distance from sensor surface of the image sensor to the one part of the living body is equal to or greater than 0.5 mm and smaller than 30 mm.

10. The biological information obtaining device according to claim 1, wherein
lens diameter of the single lenses is equal to or greater than 20 times pixel size of the image sensor,
lens magnification of the single lenses is smaller than one times, and
when s represents minimum value of photographic subject size to be resolved, when M represents lens magnification, and when N represents pixel size of the image sensor, relationship s×M>2×N is established.

11. The biological information obtaining device according to claim 1, wherein
in between the lens array and the one part of the living body,
a second-type light shield is disposed that forms a second-type light guiding path meant for guiding, in a sequential manner, light constituting the biological information from side of the lens array toward the single lenses, and
a cover glass is disposed, the one part of the living body being placed on surface of the cover glass, and
light source unit for emitting light of the predetermined wavelength onto the one part of the living body is disposed around the cover glass.

12. The biological information obtaining device according to claim 1, wherein an absorber having light absorptivity of 90% or higher at a predetermined wavelength is disposed on surface of the light shield facing the light guiding path.

13. The biological information obtaining device according to claim 1, wherein regular reflection prevention processing is performed on surface of the light shield facing the light guiding path.

14. The biological information obtaining device according to claim 1, wherein material of the light shield is made of at least either metal, or resin compound, or dielectric substance.

15. The biological information obtaining device according to claim 1, wherein the biological information indicates at least either image information related to fingerprints, or image information related to perspiration, or image information related to blood flow, or image information related to pulse wave or pulsation, or image information related to blood vessels.

16. The biological information obtaining device according to claim 1, wherein the biological information indicates at least two types of image information selected from among image information related to fingerprints, image information related to perspiration, image information related to blood flow, image information related to pulse wave or pulsation, and image information related to blood vessels.

17. The biological information obtaining device according to claim 1, wherein the biological information obtaining device is installed in a mobile device, a wearable device, or a stationary device, or is installed in an external device attachable to a stationary device.

18. A biological information obtaining method comprising performing imaging of biological information, which represents image information obtained as a result of emitting light of predetermined wavelength with respect to one part of a living body, using a lens array, which has a plurality of single lenses arranged in an array-like manner, in an image sensor via a light guiding path formed by a light shield, wherein
the light guiding path is formed to have unequal opening diameter along surface normal direction of the image sensor.

19. A wearable device comprising:
a device main body that is wearable by user; and
a biological information obtaining device that is installed in the device main body, wherein
the biological information obtaining device includes
an image sensor in which biological information, which represents image information obtained as a result of emitting light of predetermined wavelength with respect to one part of a living body, is imaged,
a lens array that
is positioned in between the image sensor and the one part of the living body,
has a plurality of single lenses arranged in an array-like manner, and
performs imaging of the biological information in the image sensor, and
a light shield that
is positioned in between the image sensor and the lens array, and
forms a light guiding path meant for imaging of the biological information, which is transmitted through each of the single lenses, in the image sensor, and
the light guiding path is formed to have unequal opening diameter along surface normal direction of the image sensor.
